# DEMANDE DE BREVET EUROPEEN

(11) **EP 1 133 956 A1**
(43) Date de publication de la demande: **19.09.2001**
(21) Numéro de dépôt: 01400691.0
(22) Date de dépôt: 15.03.2001
(51) Int. Cl.: A61F 2/28, A61C 8/00

(54) **Prothèse obturatrice maxillo-faciale**

(30) Priorité: 17.03.2000 FR 0003437
(71) Demandeur: Gourmet, René, 69008 Lyon (FR); Mai, Christian, F-69006 Lyon (FR); Bigot, Joseph, 14940 Sannerville (FR)
(72) Inventeur: Gourmet, René, 69008 Lyon (FR); Mai, Christian, F-69006 Lyon (FR); Bigot, Joseph, 14940 Sannerville (FR)
(74) Mandataire: Chambon, Gérard

(57) **Abrégé**

L'invention concerne une prothèse obturatrice maxillo-faciale (1) destinée à obturer une cavité maxillo-faciale d'un patient et comprenant une pièce obturatrice (2) prévue pour être mise en place dans la cavité à traiter et une éventuelle prothèse palatine ou palatino-dentaire (6) destinée à reconstituer la voûte palatine et éventuellement la denture au niveau de ladite cavité.

La prothèse selon l'invention est notamment remarquable en ce que ladite pièce obturatrice (2) présente une structure évidée, constituée par une paroi souple (7) dont la forme est adaptée à celle de la cavité à traiter et qui est fermée par une pièce intermédiaire (3) indépendante, aménagée pour venir au contact du bord inférieur (8) de la pièce obturatrice (2) et au contact des bords (9) délimitant l'ouverture de ladite cavité à traiter, tout en pouvant servir d'appui à l'éventuelle prothèse palatine ou palatino-dentaire (6), tandis que des organes (4; 5) sont prévus pour la fixation amovible de ladite pièce intermédiaire (3) sur le patient.

## Description

La présente invention concerne une prothèse obturatrice maxillo-faciale.

Le traitement chirurgical d'une tumeur cancéreuse maxillo-faciale consiste à pratiquer l'exérèse de cette tumeur complétée le plus souvent d'une radiothérapie.

La perte de substance maxillaire résultant de l'exérèse entraîne, selon les organes touchés et la profondeur de cette exérèse, des troubles de la phonation, de la mastication, de la déglutition ainsi qu'une altération esthétique.

Il a été envisagé d'obturer la cavité résultant de cette exérèse au moyen d'une prothèse maxillo-faciale amovible comprenant un bloc obturateur et une prothèse palatine assemblés l'un à l'autre.

Le bloc obturateur est destiné à combler ladite perte de substance et présente à cet effet une forme correspondant sensiblement à la forme que présente la cavité à traiter, ce bloc étant réalisé avec un élastomère. La prothèse palatine est destinée à reconstituer la forme du palais et à remplacer la denture dont l'ablation a été rendue nécessaire par l'exérèse, cette prothèse étant confectionnée en résine et se présentant comme une prothèse dentaire classique.

La prothèse maxillo-faciale est amovible, pour pouvoir être retirée en vue du contrôle fréquent de la cavité d'exérèse afin de permettre la détection rapide de toute récidive éventuelle.

Actuellement une prothèse obturatrice présente plusieurs inconvénients, à savoir :
- un poids important, généralement supérieur à 150 grammes, qui pose des problèmes de confort pour le patient et de maintien en position de la prothèse,
- une difficulté certaine d'insertion buccale, du fait de l'épaisseur de la prothèse et d'une éventuelle fibrose musculaire résultant d'une radiothérapie, la pose et la dépose de la prothèse étant rendues pénibles pour le patient,
- un maintien en position médiocre de la prothèse palatine en cas de patient particulièrement édenté, les mouvements de cette prothèse étant transmis au bloc obturateur, ce qui génère des irritations par ce dernier des berges de la cavité buccosinusienne et par conséquent l'inflammation desdites berges,
- un défaut d'étanchéité au niveau des fosses nasales, ce qui entraîne le reflux des liquides par les fosses nasales et une rhinolalie,
- une réalisation contraignante tant pour le patient que pour le praticien.

Il en résulte que plusieurs prothèses provisoires, adaptées aux évolutions successives du contour de la cavité à traiter, doivent être successivement mises en place au cours des premiers mois de traitement. Pour le praticien, la réalisation de la prothèse palatine implique les mêmes principes de sustentation, stabilisation et rétention que la réalisation d'une prothèse dentaire classique mais la présence du bloc obturateur complique énormément le travail dudit praticien.

La présente invention vise à remédier à ces inconvénients fondamentaux de ces types de prothèse, en fournissant une prothèse de poids réduit, propre à s'adapter aux éventuelles variations de forme de la cavité à traiter, fournissant une étanchéité satisfaisante, pouvant être parfaitement maintenue en position sans risque d'inflammation des parois délimitant cette cavité, et permettant une inspection périodique facile de ces mêmes parois.

A cet effet, l'invention propose une prothèse obturatrice maxillo-faciale destinée à obturer une cavité maxillo-faciale d'un patient et comprenant une pièce obturatrice prévue pour être mise en place dans la cavité à traiter et une éventuelle prothèse palatine ou palatino-dentaire destinée à reconstituer la voûte palatine et éventuellement la denture au niveau de ladite cavité, prothèse maxillo-faciale qui est notamment remarquable en ce que ladite pièce obturatrice présente une structure évidée, constituée par une paroi souple dont la forme est adaptée à celle de la cavité à traiter et qui est fermée par une pièce intermédiaire indépendante, aménagée pour venir au contact du bord inférieur de la pièce obturatrice et au contact des bords délimitant l'ouverture de ladite cavité à traiter, tout en pouvant servir d'appui à l'éventuelle prothèse palatine ou palatino-dentaire, tandis que des organes sont prévus pour la fixation amovible de ladite pièce intermédiaire sur le patient.

La pièce intermédiaire se présente par exemple sous la forme d'une plaque d'un matériau rigide.

La forme et la souplesse de la paroi de la pièce obturatrice avantageusement de faible épaisseur, permettent une adaptation étroite de cette pièce aux tissus délimitant la cavité à traiter, pour l'obtention d'une bonne étanchéité des fosses nasales par rapport à la cavité buccale.

La souplesse de cette paroi permet de plus à la pièce obturatrice de s'adapter aux éventuelles variations de forme des tissus délimitant la cavité à traiter.

Cette pièce obturatrice peut être réalisée de façon immédiate comme prothèse transitoire, dans les heures ou jours qui suivent l'intervention chirurgicale.

La mise en place et la dépose rapides et faciles de cette pièce sont ainsi rendues possibles.

De préférence, les organes pour la fixation amovible de la pièce intermédiaire sont du type à enclenchement réversible et selon un mode de réalisation, lesdits organes comprennent des implants destinés à coopérer avec des moyens de fixation solidaires de ladite pièce intermédiaire.

Avantageusement, les implants sont en matériau à mémoire de forme dont chacun présente, à l'état martensitique de ce matériau, une forme sensiblement en U à plat ou plié sensiblement d'équerre et, à l'état austénitique de ce matériau qui est atteint par l'évolution de la température de l'implant vers la température du corps recevant la prothèse, une forme en U évasé, les parties d'extrémité des branches latérales de cet implant se recourbant, dans cette forme, vers l'extérieur ou l'intérieur, tandis que les moyens de fixation solidaires de la pièce intermédiaire présentent chacun une forme de téton arrondi de manière à pouvoir être inséré dans l'espace délimité par la base des branches latérales et la partie reliant ces dernières de l'implant correspondant.

Les implants sont destinés à être insérés dans les tissus osseux délimitant la cavité à combler : processus palatin et tubérosité du maxillaire et on comprend que l'évasement subséquent des parties d'extrémité des branches latérales de l'implant permet un ancrage de chaque implant. La forme en U d'équerre est notamment prévue pour des cas complexes dus à l'absence de tissus osseux. Les tétons arrondis sont aménagés aux endroits adéquats sur la pièce intermédiaire qui est mise en place par enclenchement, comme précisé ci-avant.

La pièce intermédiaire peut comporter un moyen de préhension, tel qu'un plot, en vue de faciliter encore son montage ou démontage, ledit moyen de préhension étant par exemple logé dans un logement ménagé dans la prothèse palatine ou palatino-dentaire.

L'invention sera bien comprise à la lecture de la description qui va suivre d'un mode de réalisation donné à titre d'exemple non limitatif et qui se réfère aux dessins annexés dans lesquels:
- la figure 1 est une vue en perspective éclatée de différents éléments constitutifs d'une prothèse selon l'invention, à savoir: une prothèse palatino-dentaire partiellement représentée, une pièce intermédiaire et une pièce obturatrice partiellement coupée,
- la figure 2 est une vue en coupe transversale de la prothèse de la figure 1, après mise en place sur le patient,
- la figure 3 est une vue de dessous de la prothèse, au niveau de la pièce intermédiaire,
- les figures 4a et 4b sont des vues en perspective d'un implant spécifique en forme de U courbé d'équerre.

Les figures représentent, sous différents angles, une prothèse obturatrice maxillo-faciale 1, mise en place, par exemple, suite au traitement chirurgical d'une tumeur cancéreuse maxillo-faciale, par exérèse de cette tumeur ou par des opérations d'exérèse-drainage associant radiothérapie et chirurgie.

Ce traitement a conduit à l'existence d'une cavité débouchant dans la cavité buccale et au niveau des fosses nasales, que la prothèse 1 a pour fonction d'obturer. L'intervention chirurgicale a, en outre, conduit fréquemment à une ablation plus ou moins importante de la denture et la prothèse 1 vient reconstituer la voûte palatine ainsi que cette denture.

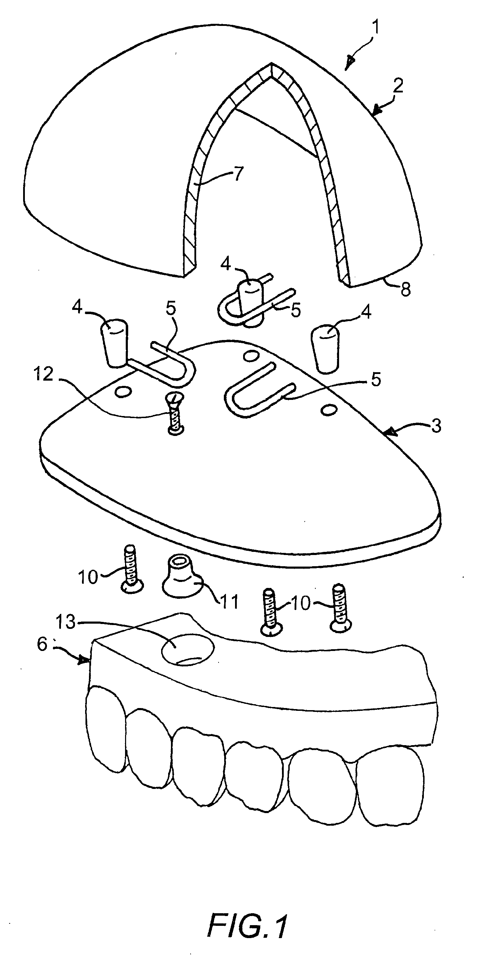

Comme le montre la figure 1, cette prothèse 1 comprend une pièce obturatrice 2, une pièce intermédiaire 3, des organes 4, 5 pour la fixation amovible de cette pièce intermédiaire 3 sur le patient et une prothèse palatino-dentaire 6.

La pièce obturatrice 2 présente une structure évidée, en coque. Cette pièce 2 a été moulée sur la base d'une empreinte de la cavité à traiter de sorte qu'elle présente extérieurement une forme adaptée à la forme de cette cavité.

La pièce 2 est, par exemple, en un élastomère de silicone et sa paroi 7 présente une relativement faible épaisseur, ce qui confère à cette paroi 7 une certaine souplesse élastique.

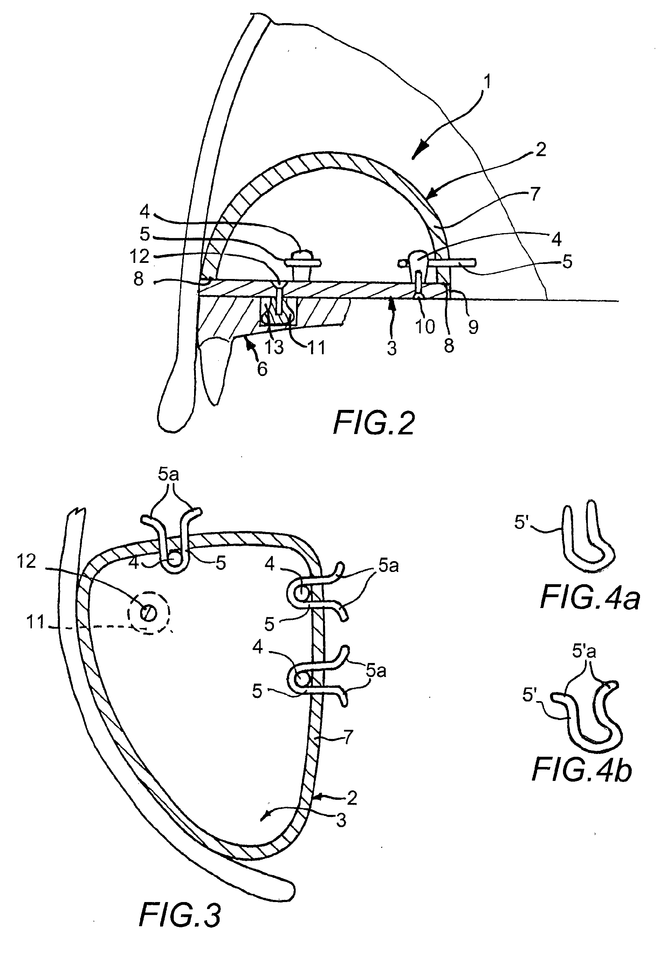

Comme le montre la figure 2, le bord inférieur 8 de la pièce obturatrice 2 est aménagé de manière à s'interrompre légèrement en retrait des bords 9 délimitant l'ouverture de la cavité à traiter après mise en place de ladite pièce 2 dans cette cavité.

La pièce intermédiaire 3 est en matière synthétique rigide du type de celle couramment utilisée pour réaliser des prothèses dentaires.

Cette pièce 3 a la forme d'une plaque et présente un contour qui correspond à celui de l'ouverture de la cavité à traiter de telle sorte que lorsqu'elle est mise en place comme le montre la figure 2, elle vient au contact du bord inférieur 8 de la pièce obturatrice 2 et elle est affleurée avec les bords 9 de ladite cavité.

La pièce 3 peut également, dans des cas particuliers, être fixée directement sur la prothèse palatino-dentaire 6 (au moyen par exemple d'agrafes ou autres).

La pièce 3 comporte des tétons 4 de forme arrondie, fixés sur elle au moyen de vis 10 et un plot de préhension 11 fixé par une vis 12 qui permet sa saisie en vue de sa mise en place sur le patient, ou de son retrait.

Les organes pour la fixation amovible de la pièce 3 comprennent, outre les tétons 4 déjà décrits, des implants 5 en matériau à mémoire de forme. Ainsi que cela apparaît par comparaison des figures 1 et 3, chaque implant 5 présente, à l'état martensitique de son matériau, une forme en U et, à l'état austénitique de ce matériau qui est atteint par l'évolution de la température de l'implant 5 vers la température du corps recevant la prothèse, une forme en U évasé, les parties d'extrémité 5a des branches latérales de l'implant 5 se recourbant, dans ce mode de réalisation, vers l'extérieur dudit implant 5 (le recourbement pouvant selon les cas s'effectuer vers l'intérieur).

La prothèse palatino-dentaire 6 est, quant à elle, de type classique sinon qu'elle comporte un logement 13 pour recevoir le plot de préhension 11.

En pratique, les implants 5 sont implantés par insertion dans les tissus osseux qui délimitent la cavité. Cette insertion est réalisée jusqu'à ce que la base des branches latérales et la partie reliant ces dernières de chaque implant 5 fassent encore suffisamment saillie à l'intérieur de la cavité à traiter pour que chaque implant 5 délimite un espace propre à recevoir avec frottements le téton 4 correspondant de la pièce 3, ces frottements créant un assemblage par enclenchement réversible.

L'évasement des parties d'extrémité 5a des branches latérales de chaque implant 5 permet un ancrage solide aux tissus osseux.

Les figures 4a et 4b montrent des implants particuliers 5' en forme chacun de U mais plié sensiblement d'équerre (c'est-à-dire ne se présentant pas à plat ou dans un plan comme dans le mode de réalisation représenté sur les figures 1 à 3) pour des fixations plus délicates ou par exemple pour favoriser la réalisation d'une prothèse mandibulaire totale.

La figure 4a montre un implant 5' dans sa forme initiale et la figure 4b montre le même implant 5' en forme finale avec ses parties d'extrémité 5'a recourbées.

C'est après l'insertion des implants 5 que la pièce 2, préalablement moulée à la forme de la cavité à traiter, est disposée dans cette cavité aux fins de repérer les emplacement desdits implants 5 pour percer des ajours à l'emporte-pièce au travers de sa paroi 7, en regard de chacun des implants 5.

Les positions des implants 5 sont ensuite repérées par rapport à la pièce 3, puis cette pièce 3 est percée d'ajours destinés à recevoir les vis 10. Les tétons 4 sont mis en place sur le patient, par enclenchement au travers des implants 5.

La prothèse palatino-dentaire 6 est à son tour fixée classiquement mais de manière à prendre appui contre la pièce 3, le plot 11 étant engagé dans le logement 13.

La forme et la souplesse de la pièce 2 permettent une adaptation parfaite de celle-ci aux tissus délimitant la cavité à traiter et une adaptation de cette pièce 2 aux éventuelles variations de forme desdits tissus.

La pièce 2, par sa souplesse et par son indépendance par rapport à la pièce 3, est facile à mettre en place.

La légèreté de cette pièce 2, grâce à sa structure évidée, permet un confort du patient et une parfaite immobilisation de cette pièce 2 par rapport aux tissus.

La pièce 3 vient fermer la cavité délimitée intérieurement par la pièce 2, en venant, comme déjà dit, au contact des tissus délimitant l'ouverture de la cavité ainsi qu'au contact du bord 8 de la pièce 2. Ladite pièce 3 complète ainsi l'étanchéité de la cavité à traiter par rapport à la cavité buccale.

Le retrait de la pièce 3, par extraction des plots 4 en dehors des implants 5, est rendu facile et rapide grâce au plot 11 de sorte que l'opération d'inspection périodique des tissus délimitant la cavité est elle-même facile à réaliser.

En outre, l'indépendance de la prothèse palatino-dentaire 6 par rapport à la pièce 3 permet qu'aucun des éventuels mouvements de cette prothèse ne soit transmis à cette pièce 3 ou à la pièce 2. Aucun frottement répété n'est créé entre, d'une part, la pièce 3 et la pièce 2 et, d'autre part, les tissus contre lesquels cette pièce 3 et cette pièce 2 prennent appui.

Cette prothèse palatine dentaire 6 vient de plus parfaire l'étanchéité de la prothèse maxillo-faciale 1.

L'invention fournit ainsi une prothèse obturatrice maxillo-faciale permettant de remédier à l'ensemble des inconvénients des prothèses homologues de la technique antérieure, par sa légèreté, son étanchéité et sa facilité de mise en place et de retrait.

## Revendications

1. Prothèse obturatrice maxillo-faciale (1) destinée à obturer une cavité maxillo-faciale d'un patient et comprenant une pièce obturatrice (2) prévue pour être mise en place dans la cavité à traiter et une éventuelle prothèse palatine ou palatino-dentaire (6) destinée à reconstituer la voûte palatine et éventuellement la denture au niveau de ladite cavité, prothèse **caractérisée en ce que** ladite pièce obturatrice (2) présente une structure évidée, constituée par une paroi souple (7) dont la forme est adaptée à celle de la cavité à traiter et qui est fermée par une pièce intermédiaire (3) indépendante, aménagée pour venir au contact du bord inférieur (8) de la pièce obturatrice (2) et au contact des bords (9) délimitant l'ouverture de ladite cavité à traiter, tout en pouvant servir d'appui à l'éventuelle prothèse palatine ou palatino-dentaire (6), tandis que des organes (4; 5,5') sont prévus pour la fixation amovible de ladite pièce intermédiaire (3) sur le patient.

2. Prothèse selon la revendication 1, **caractérisée en ce que** la pièce intermédiaire (3) se présente sous la forme d'une plaque d'un matériau rigide.

3. Prothèse selon l'une des revendications 1 et 2, **caractérisée en ce que** lesdits organes (4; 5,5') pour la fixation amovible de la pièce intermédiaire (3) sont du type à enclenchement réversible.

4. Prothèse selon la revendication 3, **caractérisée en ce que** les organes (4; 5,5') pour la fixation amovible de la pièce intermédiaire (3), comprennent des implants (5,5') destinés à coopérer avec des moyens de fixation (4) solidaires de ladite pièce intermédiaire (3).

5. Prothèse selon la revendication 4, **caractérisée en ce que** les implants (5,5') sont en matériau à mémoire de forme dont chacun présente, à l'état martensitique de ce matériau, une forme sensiblement en U à plat ou plié sensiblement d'équerre et, à l'état austénitique de ce matériau qui est atteint par l'évolution de la température de l'implant vers la température du corps recevant la prothèse (1), une forme en U évasé, les parties d'extrémité (5a,5'a) des branches latérales de cet implant (5,5') se recourbant, dans cette forme, vers l'extérieur ou l'intérieur, tandis que les moyens de fixation solidaires de la pièce intermédiaire (3) présentent chacun une forme de téton (4) arrondi de manière à pouvoir être inséré dans l'espace délimité par la base des branches latérales et la partie reliant ces dernières de l'implant (5,5') correspondant.

6. Prothèse selon l'une des revendications 1 à 5, **caractérisée en ce que** la pièce intermédiaire (3) comporte un moyen de préhension (11) tel qu'un plot.

7. Prothèse selon la revendication 6, avec prothèse palatine ou palatino-dentaire (6), **caractérisée en ce que** le moyen de préhension (11) de la pièce intermédiaire (3) est logé dans un logement (13) ménagé dans ladite prothèse palatine ou palatino-dentaire (6).
